# EUROPEAN PATENT APPLICATION

(11) **EP 3 669 822 A1**
(43) Date of publication of application: **24.06.2020**
(21) Application number: 18849214.4
(22) Date of filing: 15.08.2018
(51) Int. Cl.: A61C 17/02, A61C 17/16

(54) **SPRAYER FOR VISUAL ORAL IRRIGATOR, AND VISUAL ORAL IRRIGATOR**

(30) Priority: 19.08.2017 CN 201710714595
(71) Applicant: Zhou, Xing, Guangdong 510663 (CN)
(72) Inventor: Zhou, Xing, Guangdong 510663 (CN)
(74) Representative: Boult Wade Tennant LLP
(86) International application number: PCT/CN2018/100718
(87) International publication number: WO 2019/037643

(57) **Abstract**

A visual oral irrigator of the invention includes an oral irrigator, an oral observation instrument and a connecting mechanism. A water outlet of a sprayer is within the field of view of an observation system of the oral observation instrument. A pressure vessel includes a pressurizing device, a housing and a fluid containing space. The pressure of water in the pressure vessel can be increased through the pressurizing device, and a control switch is turned on to enable the water to be sprayed from the sprayer of a spray lance, so that the teeth or the oral cavity can be cleaned visually, and the cleaning process can be shot and photographed in real time. By adopting a detachable design mode for the visual oral irrigator of the invention, both the sprayer and a water tank can be conveniently removed and then cleaned, so that the using process is more sanitary and convenient.

## Description

### FIELD OF THE INVENTION

The invention relates to a tool for oral cleaning, and particularly relates to a visual oral irrigator for cleaning teeth, interdental spaces and oral cavities.

### BACKGROUND OF THE INVENTION

Due to reasons of age, pathology, and the like, gingival atrophy and interdental expansion are prone to occur, which is easy to cause food residues. If the food residue cannot be cleaned in time, on the one hand, it will cause bad breath, and on the other hand, it is easy to cause various dental diseases and oral diseases, especially periodontitis.

In view of this situation, various dental flosses, interdental brushes, water flosses and other products have been developed on the market for users to choose. Although there are dental flosses, interdental brushes and other products with various structures and different specifications on the market, the existing products generally have the defects that they are difficult in observing the tooth gap. Especially, when the tooth gap between a third molar and a second molar, or the tooth gap between a second molar and a first molar, or the tooth gap between a first molar and a second premolar is cleaned, because the tooth gap to be cleaned is positioned deep in the mouth, the light at the part to be cleaned is very dark, the tooth gap to be cleaned cannot be seen clearly, and a user can only perform the cleaning operation by feeling, which easily causes gingival bleeding or damage.

In order to overcome the above technical disadvantages, the applicant discloses a visual oral irrigator for performing interdental cleaning visually in the patent application "Visual Oral Irrigator" (Application No.). The present application is a further improvement on the sprayer.

### SUMMARY

The invention provides a sprayer for a visual oral irrigator. A sprayer 11 includes a nozzle 11-1 and a connecting mechanism 11-2;
A. the nozzle 11-1 includes a water inlet 11-11, a channel 11-12 and a water outlet 11-13; the water inlet 11-11 is connected to an outlet 12-11 of a fluid channel 12-1 of a spray lance 12 of an oral irrigator 100 of a visual oral irrigator 900; a fluid in the oral irrigator 100 flows through the outlet 12-11 of the fluid channel 12-1 of the spray lance 12, then enters the water inlet 11-11, flows through the channel 11-12 and then is sprayed from the water outlet 11-13;
B. the nozzle 11-1 is detachably mounted on a housing 21 of an oral observation instrument 200 of the visual oral irrigator 900 through the connecting mechanism 11-2 to realize connection between the sprayer 11 and the spray lance 12.

The sprayer for a visual oral irrigator of the invention is connected to the spray lance 12 in a detachable mounting mode, so that after use, the sprayer 11 can be removed from the oral observation instrument 200 and then cleaned and replaced, which facilitates cleaning and makes the using process more sanitary.

The water outlet 11-13 is within the field of view of an observation system 24 of the oral observation instrument 200. The water outlet 11-13 is within the field of view of the observation system 24 of the oral observation instrument 200 to ensure that in a using process, the water outlet 11-13 can be directly aligned to the tooth gap to be cleaned, after a switch is turned on, the sprayed fluid can directly enter the tooth gap to rinse food residues, and simultaneously, the position of the water outlet 11-13 and the flow rate, moving direction and moving speed of the fluid when sprayed from the water outlet 11-13 can be observed in real time, thereby really realizing visual and accurate operation on the part to be cleaned.

The water outlet 11-13 has a smooth end surface 11-12-1. Because the sprayer 11 often needs to slide against the surface of the teeth or gingivae in the using process, the smooth end surface is designed to ensure that there is no accidentally damage to the teeth or gingivae in the using process, and the using process is safer and more comfortable. According to different shapes of the nozzle 11-1, the end surface 11-12-1 may also be a smooth end surface in a flat shape or a spherical surface or other shapes correspondingly.

The nozzle 11-1 has a radian 11-14 meeting the requirements of a human oral cavity. Especially, when it is necessary to clean the molars on the rear side along the outer side of the teeth, the radian 11-14 allows the sprayer 11 to smoothly reach the positions of the molars, so that the using process is more comfortable.

The water outlet 11-13 has different shapes, such as a conical shape, an elliptical shape, a flat shape or a spherical shape. The water outlets 11-13 of different shapes can spray water of different shapes. For example, a conical water outlet 11-13 may spray a single water column or a plurality of water columns, and a spherical water outlet 11-13 may form a water flow in a shower shape. Furthermore, the water outlets 11-13 of different shapes can spray water columns of different shapes, such as a linear shape or a horn shape. A user can select different outlet shapes according to the shape and area of the part to be cleaned. For example, the user can select a conical outlet for cleaning the tooth gap, a flat outlet for cleaning the joint between the teeth and the gingivae, and a spherical outlet for cleaning the body of tongue, so that the using process is more convenient. Herein, the applicant only enumerates several shapes of the water outlets, and those skilled in the art may design the water outlets 11-13 of other various shapes according to needs without departing from the scope of protection of the present application.

The connecting mechanism 11-2 includes a positioning block 31; the positioning block 31 is of an inverted T-shaped structure; the housing 21 of the oral observation instrument 200 is provided with a positioning groove 32 and a clamping block 33 capable of being connected to the connecting mechanism 11-2; and the positioning block 31 on the connecting mechanism 11-2 can be embedded in the positioning groove 32, and the clamping block 33 can prevent the connecting mechanism 11-2 from sliding backward, so that the sprayer 11 is fixed on the oral observation instrument 200 to realize connection between the sprayer 11 and the spray lance 12.

The visual oral irrigator of the invention is characterized in that the oral irrigator 100 of the visual oral irrigator 900 includes the sprayer 11.

The visual oral irrigator 900 includes an oral irrigator 100, an oral observation instrument 200 and a connecting mechanism 300;
A. the oral irrigator 100 includes a sprayer 11, a spray lance 12, a control switch 13, a pressure vessel 14, a connecting pipe 15 and a connector 16; the sprayer 11 is detachably disposed at a front end of the spray lance 12; the control switch 13 can control the spraying of the fluid in the spray lance 12; the fluid in the pressure vessel 14 is connected to the spray lance 12 through the connecting pipe 15 and the connector 16;
B. the oral observation instrument 200 includes a housing 21, a power system 22, an illuminating system 23, an observation system 24, a circuit system 25 and a switch 26; the illuminating system 23, the observation system 24, the circuit system 25 and the power system 22 are mounted in the housing 21, and the switch 26 is mounted on the housing 21; the illuminating system 23, the observation system 24, the power system 22 and the switch 26 are connected together through the circuit system 25;
C. the oral irrigator 100 is mounted on the oral observation instrument 200 through the connecting mechanism 300.

When the visual oral irrigator of the invention is used, the water outlet 11-13 of the sprayer 11 can be directly aligned to the part to be cleaned, such as the tooth gap, the control switch 13 is turned on, and the pressurized fluid in the pressure vessel 14 is sprayed from the water outlet 11-13 to realize visual and accurate cleaning on the part to be cleaned.

The pressure vessel 14 of the oral irrigator 100 includes a pressurizing device 14-1 and a water tank 14-12; the water tank 14-12 includes a housing 14-2 and a fluid containing space 14-3; and the pressurizing device 14-1 can pressurize the fluid in the water tank 14-12.

The pressurizing device 14-1 may be a mechanical pressurizing device or an electric pressurizing device. In the invention, the pressurizing device 14-1 is an electric pressurizing device 142. The electric pressurizing device 142 can realize automatic pressurization by just switching on a power source and keep the pressure stable, so that the using process is more convenient. When the pressurizing device 14-1 is the electric pressurizing device 142, the power system 22 may be provided with a pressurizing device 22-1 to increase the voltage of the power system 22, thereby better driving the electric pressurizing device 142 to work.

The water tank 14-12 is detachably mounted on the connecting pipe 15. The connecting pipe 15 is provided with a connecting seat 15-3, and the water tank 14-12 is detachably connected to the connecting pipe 15 through the connecting seat 15-3. By virtue of a detachable connection mode, the water tank 14-12 can be conveniently removed from the connecting pipe 15 and then cleaned in the using process, so that the using process is more sanitary.

The water tank 14-12 is connected to the connecting pipe 15 in a mode of threaded connection, or a mode of concave-convex clamping connection or a mode of interference fit connection. Herein, the applicant only enumerates the above three detachable connection modes, and those skilled in the art may design the connection modes of other various structures according to needs without departing from the scope of protection of the present application.

The observation system 24 is a camera system 24-1, and the camera system 24-1 includes a camera 24-1-1, a data processing and output system 24-1-2, a circuit system 25 and a power system 22.

Video data output by the data processing and output system 24-1-2 of the camera system 24-1 can be displayed on a display 24-1-3 in a mode of wired connection or wireless connection, and the display 24-1-3 includes a smart phone 24-1-31, or a computer 24-1-32, or a liquid crystal display 24-1-33, or a television 24-1-34.

The illuminating system 23 is disposed at the periphery of the camera 24-1-1. In order to increase illumination, more LED lamps may also be disposed on the housing 21 of the oral observation instrument 200 so as to adjust the illumination brightness.

A front end 900-1 of the visual oral irrigator 900 is connected to a main body 900-2 of the visual oral irrigator 900 through a foldable connecting mechanism 900-4.

The foldable connecting mechanism 900-4 is a rotating shaft type moving mechanism or a concave-convex clamping mechanism.

The foldable connecting mechanism 900-4 is a rotating shaft type connecting mechanism, the foldable connecting mechanism 900-4 includes a rotating shaft 900-4-1 and a rotating shaft hole 900-4-2, and the rotating shaft 900-4-1 can move in the rotating shaft hole 900-4-2.

Of course, those skilled in the art may design the folding modes of other various structures according to needs without departing from the scope of protection of the present application.

A front end 200-1 of the oral observation instrument 200 of the visual oral irrigator 900 is connected to a main body 200-2 of the oral observation instrument 200 through a foldable connecting mechanism 900-4. Because a circuit 25-1 of the front end 200-1 of the oral observation instrument 200 is connected to a circuit 25-2 of the main body 200-2 of the oral observation instrument 200 through a bendable flexible circuit, after use, the front end 200-1 of the oral observation instrument 200 is rotated around the rotating shaft 900-4-1, and then, the front end 200-1 of the oral observation instrument 200 can be folded relative to the main body 200-2 of the oral observation instrument 200 to facilitate storage and carrying. When the oral observation instrument 200 needs to be used, the front end 200-1 of the oral observation instrument 200 is reversely rotated around the rotating shaft 900-4-1, so that the oral observation instrument 200 can be unfolded.

A front end 100-1 of the oral irrigator 100 of the visual oral irrigator 900 is connected to a main body 100-2 of the oral irrigator 100 through a foldable connecting mechanism 900-4. When the front end 100-1 of the oral irrigator 100 rotates around the rotating shaft 900-4-1 of the foldable connecting mechanism 900-4, the front end 100-1 of the oral irrigator 100 may be unfolded or folded relative to the main body 100-2 of the oral irrigator 100. A water path of the front end 100-1 of the oral irrigator 100 is connected to a water path of the main body 100-2 of the oral irrigator 100 through a bevel connecting component 100-3. The bevel connecting component 100-3 does not need to be repeatedly folded like a hose in a folding process, so that the fatigue fracture of the hose caused by repeated bending can be effectively avoided, thereby effectively prolonging the service life of the product.

In use, firstly, the front end 900-1 of the visual oral irrigator 900 is rotated around the foldable connecting mechanism 900-4 to unfold the visual oral irrigator 900, and then, the sprayer 11 is connected to the housing 21 of the oral observation instrument 200 through the connecting mechanism 11-2 to realize connection between the sprayer 11 and the spray lance 12. The oral observation instrument 200 is started, the water outlet 11-13 of the sprayer 11 is aligned to the tooth gap to be cleaned, the control switch 13 is turned on, and then the pressurized water can be sprayed from the water outlet 11-13 to accurately clean the part to be cleaned. When the water tank 14-2 needs to be cleaned, the water tank 14-2 is removed from the connecting seat 15-3 and then is cleaned. After use, the sprayer 11 and the water tank 14-2 are removed and then cleaned thoroughly, the front end 900-1 of the visual oral irrigator 900 is rotated around the foldable connecting mechanism 900-4 to fold the visual oral irrigator 900 for storage.

The visual oral irrigator 900 of the invention includes an oral irrigator 100, an oral observation instrument 200 and a connecting mechanism 300. The oral irrigator 100 includes a sprayer 11, a spray lance 12, a control switch 13, a pressure vessel 14, a connecting pipe 15 and a connector 16. The sprayer 11 is disposed at the front end of the spray lance 12. The control switch 13 is disposed on the spray lance 12. The fluid in the pressure vessel 14 is connected to the spray lance 12 through the connecting pipe 15 and the connector 16. The oral irrigator 100 is mounted on the oral observation instrument 200 through the connecting mechanism 300. The sprayer 11 of the spray lance 12 is within the field of view of the observation system 24 of the oral observation instrument 200. The pressure vessel 14 of the oral irrigator 100 includes a pressurizing device 14-1 and a water tank 14-12. The fluid pressure is increased through the pressurizing device 14-1, and the control switch 13 is turned on to enable the water to be sprayed from the sprayer 11 of the spray lance 12, so that the teeth and the oral cavity can be cleaned visually, and the cleaning process can be recorded by shooting and photographing.

Because the water outlet 11-13 of the visual oral irrigator 900 of the invention is within the field of view of the observation system 24, the position of the water outlet 11-13 and the flow rate, moving direction and moving speed of the fluid when sprayed from the water outlet 11-13 can be observed in real time, so as to really realize visual and accurate operation on the part to be cleaned. Furthermore, by adopting a detachable design mode for the visual oral irrigator of the invention, both the sprayer 11 and the water tank 14-2 can be conveniently removed and then cleaned, so that the using process is more sanitary and convenient.

### BRIEF DESCRIPTION OF THE DRAWINGS

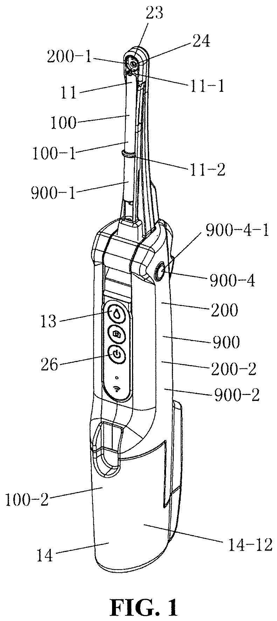

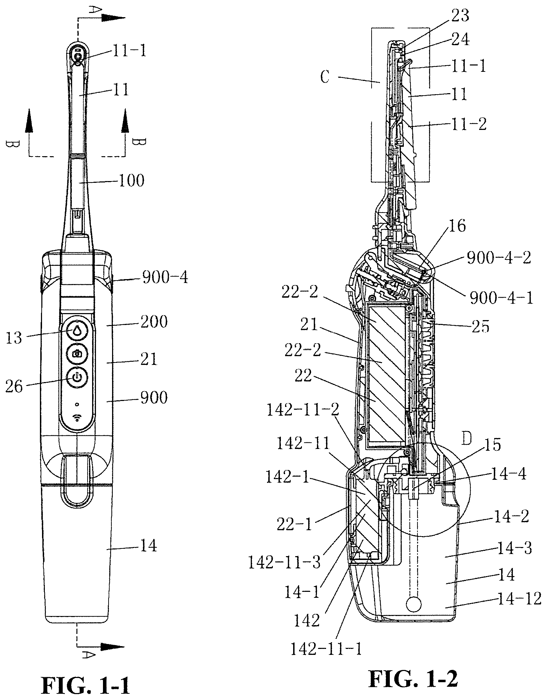

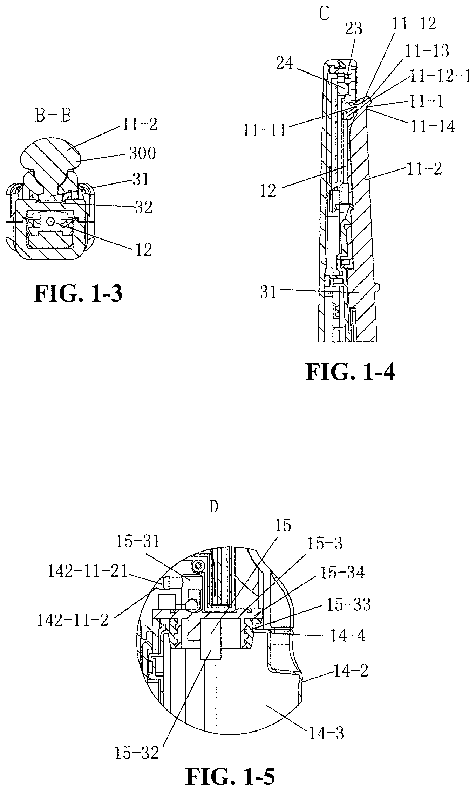

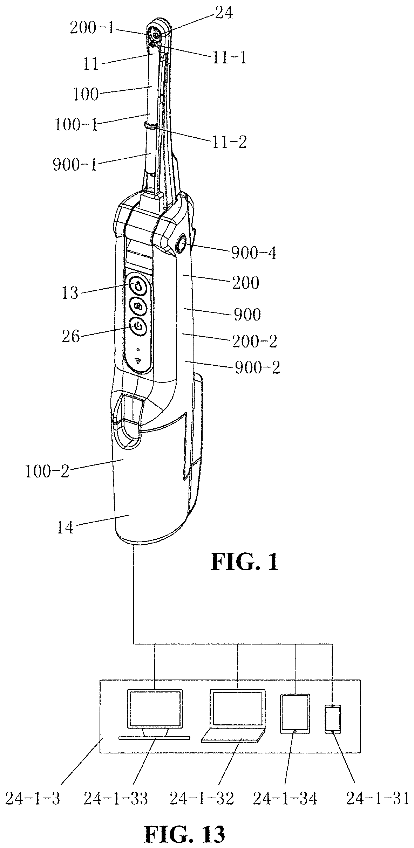

FIG. 1 is a schematic perspective view of a visual oral irrigator of the invention.
FIG. 1-1 is a front view of FIG. 1.
FIG. 1-2 is a cross-sectional view along A-A of FIG. 1-1.
FIG. 1-3 is a cross-sectional view along B-B of FIG. 1-1.
FIG. 1-4 is a partial enlarged view of a position C of FIG. 1-1.
FIG. 1-5 is a partial enlarged view of a position D of FIG. 1-1.
FIG. 2 is a front view of a visual oral irrigator of the invention with a sprayer removed.
FIG. 2-1 is a cross-sectional view along E-E of FIG. 2.
FIG. 2-2 is a cross-sectional view along F-F of FIG. 2.

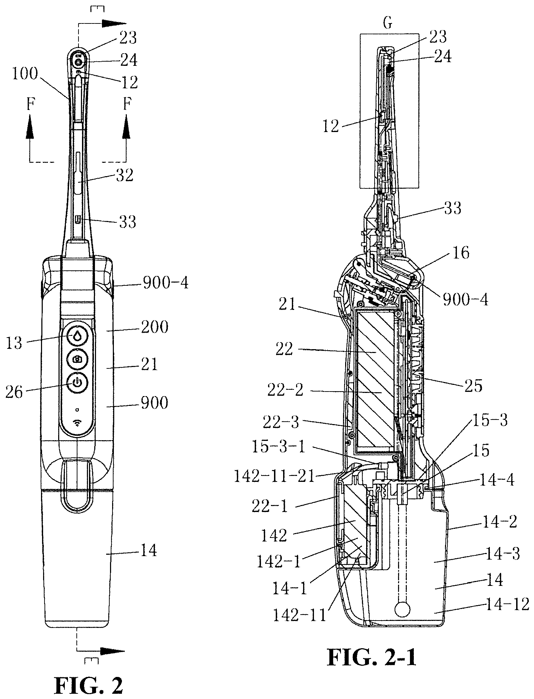

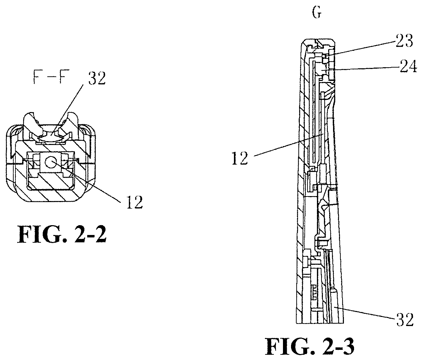

FIG. 2-3 is a partial enlarged view of a position G of FIG. 2.

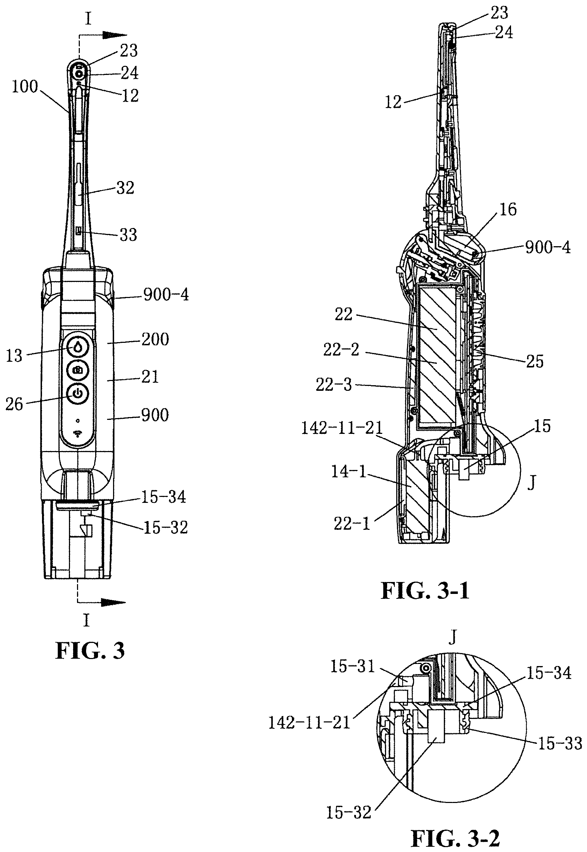

FIG. 3 is a front view of a visual oral irrigator of the invention with a water tank removed.
FIG. 3-1 is a cross-sectional view along H-H of FIG. 3.
FIG. 3-2 is a partial enlarged view of a position I of FIG. 3.

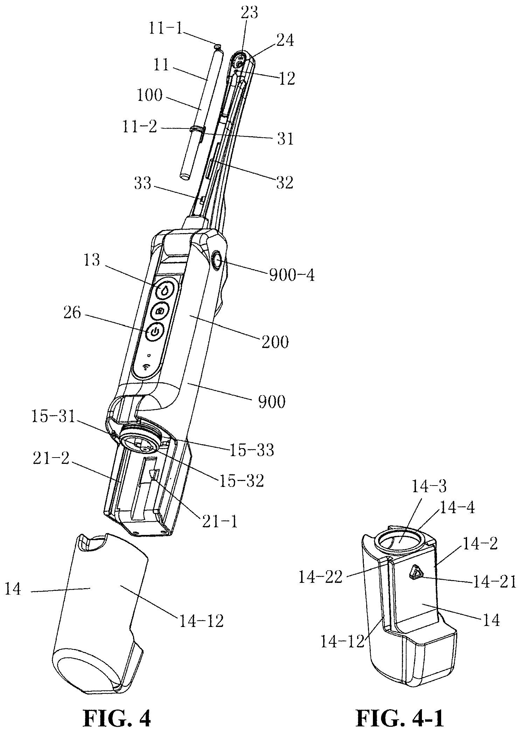

FIG. 4 is an exploded view of FIG. 1.
FIG. 4-1 is a schematic perspective view of a water tank of FIG. 4.

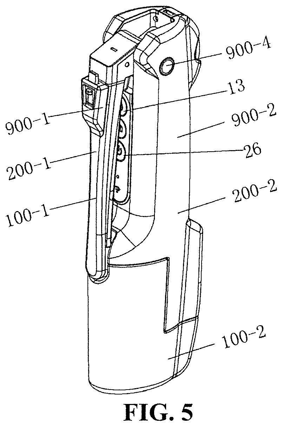

FIG. 5 is a schematic perspective view of a visual oral irrigator of the invention in a folded state.

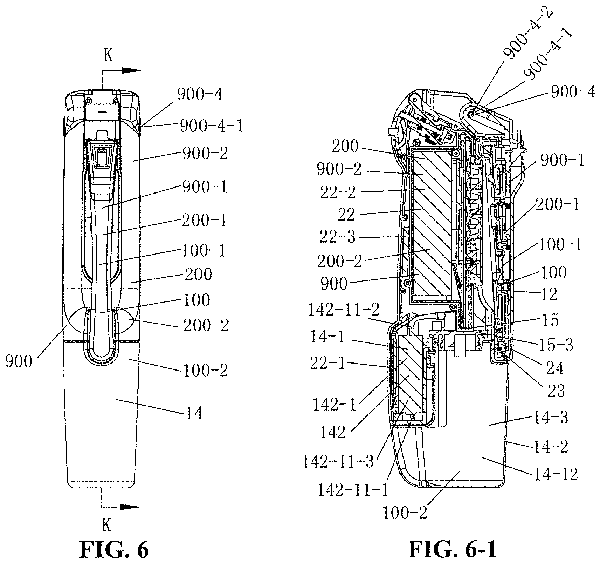

FIG. 6 is a front view of FIG. 5.
FIG. 6-1 is a cross-sectional view along J-J of FIG. 6.

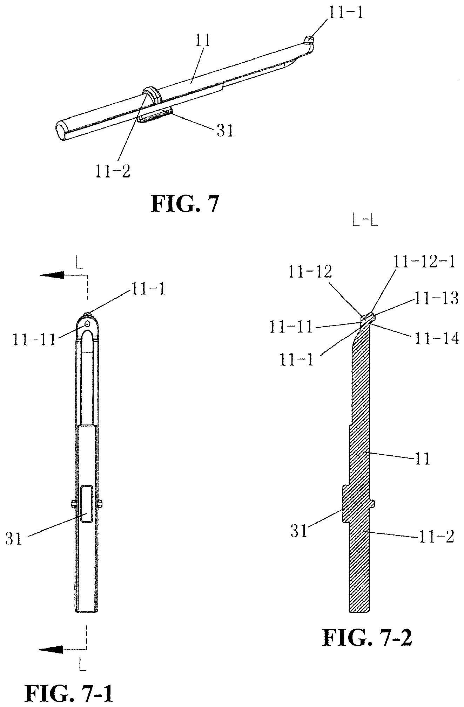

FIG. 7 is a schematic perspective view of a conical nozzle.
FIG. 7-1 is a bottom view of FIG. 7.
FIG. 7-2 is a cross-sectional view along K-K of FIG. 7-1.
FIG. 7-3 is a cross-sectional view along L-L of FIG. 7-1.

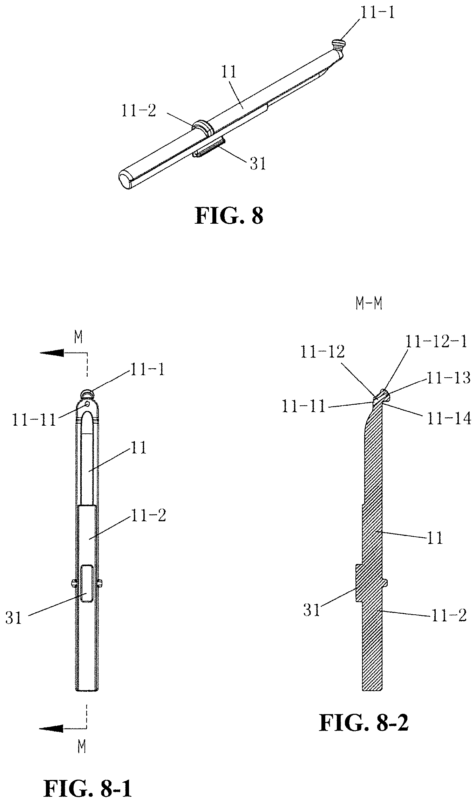

FIG. 8 is a schematic perspective view of a spherical nozzle.
FIG. 8-1 is a bottom view of FIG. 8.
FIG. 8-2 is a cross-sectional view along M-M of FIG. 8-1.

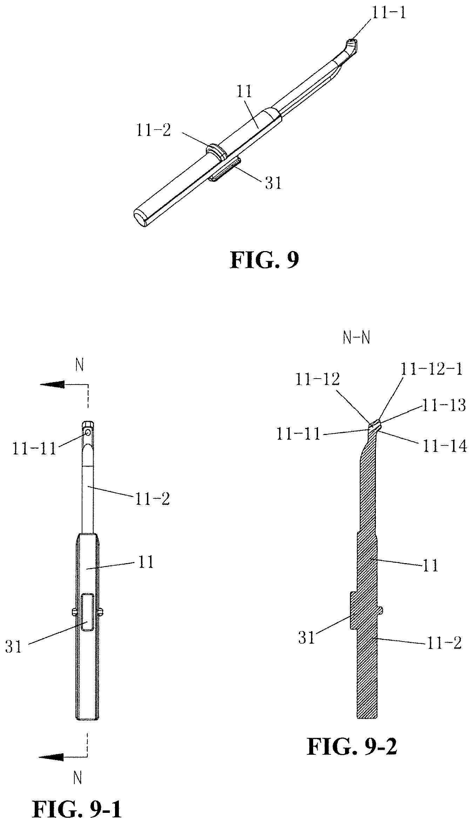

FIG. 9 is a schematic perspective view of a flat nozzle.
FIG. 9-1 is a bottom view of FIG. 9.
FIG. 9-2 is a cross-sectional view along N-N of FIG. 9-1.

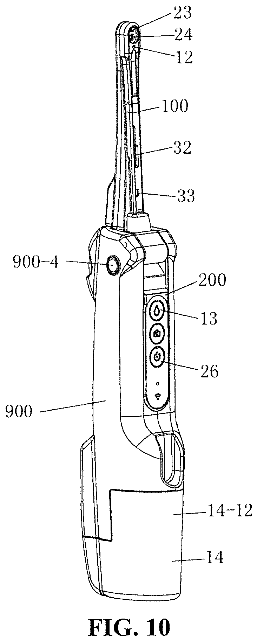

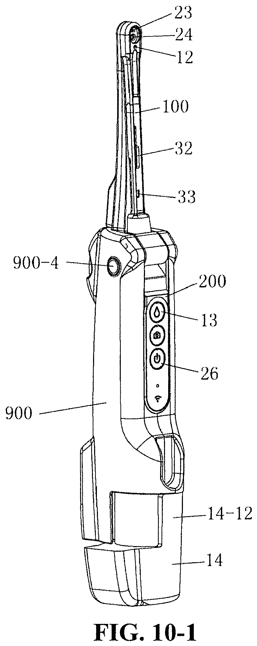

FIG. 10 is a schematic perspective view of a visual oral irrigator with a threaded connection type water tank.
FIG. 10-1 is a schematic perspective view of a water tank of FIG. 10 after rotating.

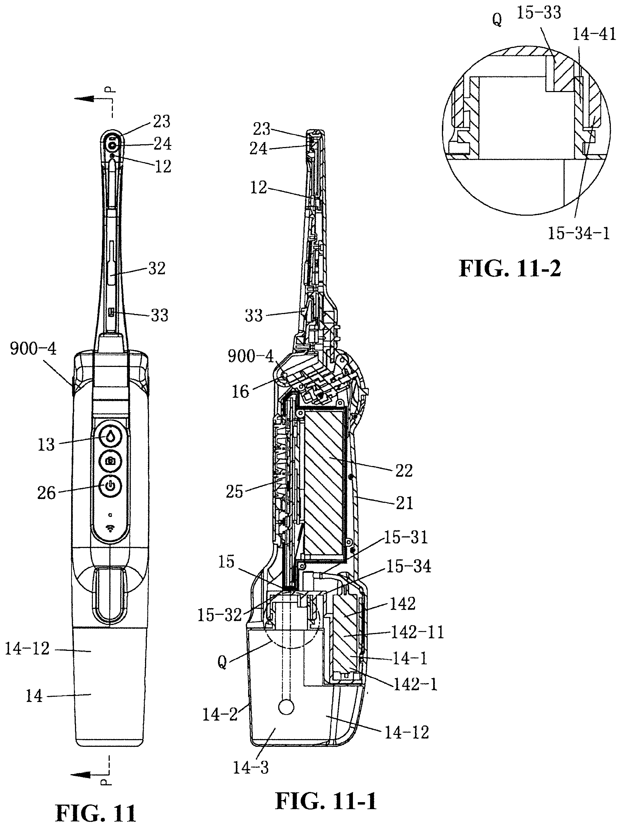

FIG. 11 is a front view of FIG. 10.
FIG. 11-1 is a cross-sectional view along P-P of FIG. 11.
FIG. 11-2 is a partial enlarged view of a position Q of FIG. 11-1.

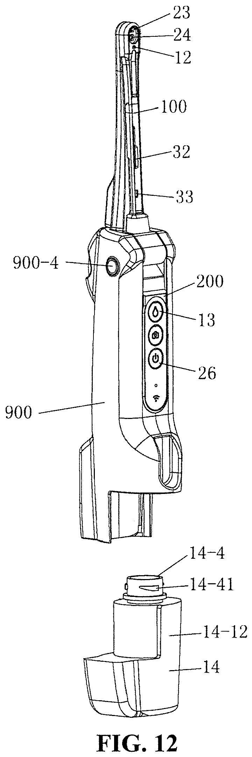

FIG. 12 is a schematic perspective view of FIG. 10 after a water tank is removed.
FIG. 13 is a schematic view of a visual oral irrigator of the invention connected to a display in a wired mode.

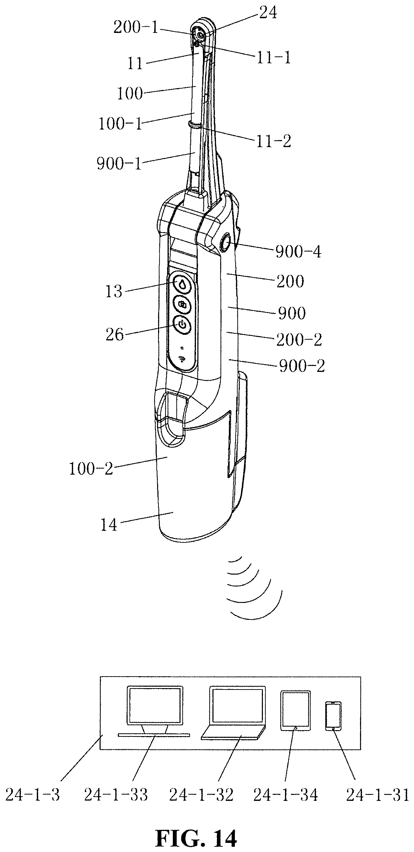

FIG. 14 is a schematic view of a visual oral irrigator of the invention connected to a display in a wireless mode.

**In the above drawings:**
oral irrigator 100, oral observation instrument 200, connecting mechanism 300, visual oral irrigator 900 of the invention;
front end 100-1 of oral irrigator, main body 100-2 of oral irrigator;
front end 200-1 of oral observation instrument, main body 200-2 of oral observation instrument;
front end 900-1 of visual oral irrigator of the invention, main body 900-2 of visual oral irrigator of the invention, foldable connecting mechanism 900-4;
rotating shaft 900-4-1, rotating shaft hole 900-4-2.

**On oral irrigator:**
sprayer 11, spray lance 12, control switch 13, pressure vessel 14, connecting pipe 15, connector 16, base 17;
nozzle 11-1, connecting mechanism 11-2;
water inlet 11-11, channel 11-12, water outlet 11-13, radian 11-14, smooth end surface 11-12-1;
pressurizing device 14-1 of pressure vessel, water tank 14-12, housing 14-2 of pressure vessel, fluid containing space 14-3 of pressure vessel, water filling hole 14-4, wedge-shaped positioning block 14-21, slide groove 14-22, internal thread 14-41;
connecting seat 15-3, gas inlet 15-31, water outlet 15-32, seal ring 15-33, base 15-34, external thread 15-34-1;
electric pressurizing device 142;
electric air pressurizing device 142-1;
electric air compressor 142-11, gas inlet 142-11-1, gas filling pipe 142-11-2, gas filling hole 142-11-21, air compressor 142-11-3.

**On oral observation instrument:**
housing 21, power system 22, illuminating system 23, observation system 24, circuit system 25, switch 26;
positioning groove 21-1, slide rail 21-2;
pressurizing device 22-1, power source 22-2, connecting mechanism 22-3;
camera system 24-1;
camera 24-1-1, data processing and output system 24-1-2, display 24-1-3;
smart phone 24-1-31, computer 24-1-32, liquid crystal display/television 24-1-33, tablet computer 24-1-34;
circuit 25-1 of front end of oral observation instrument, circuit 25-2 of main body of oral observation instrument.

**On connecting mechanism:**
positioning block 31 for mounting oral irrigator on oral observation instrument, positioning groove 32 for mounting oral irrigator on oral observation instrument, clamping block 33 for mounting oral irrigator on oral observation instrument.

### DESCRIPTION OF EMBODIMENTS

### Embodiment 1: sprayer for visual oral irrigator of the invention

Referring to FIG. 1 to FIG. 9, in the present embodiment, a sprayer 11 for a visual oral irrigator includes a nozzle 11-1 and a connecting mechanism 11-2.

The nozzle 11-1 includes a water inlet 11-11, a channel 11-12 and a water outlet 11-13. The water inlet 11-11 is connected to an outlet 12-11 of a fluid channel 12-1 of a spray lance 12 of an oral irrigator 100 of a visual oral irrigator 900. A fluid in the oral irrigator 100 flows through the outlet 12-11 of the fluid channel 12-1 of the spray lance 12, then enters the water inlet 11-11, flows through the channel 11-12 and then is sprayed from the water outlet 11-13.

The nozzle 11-1 is detachably mounted on a housing 21 of an oral observation instrument 200 of the visual oral irrigator 900 through the connecting mechanism 11-2 to realize connection between the sprayer 11 and the spray lance 12.

The water outlet 11-13 is within the field of view of an observation system 24 of the oral observation instrument 200. The water outlet 11-13 is within the field of view of the observation system 24 of the oral observation instrument 200 to ensure that in a using process, the water outlet 11-13 can be directly aligned to the tooth gap to be cleaned, and after a switch is turned on, the sprayed fluid can directly enter the tooth gap to rinse food residues, thereby really realizing accurate cleaning on the tooth gap in a visual state.

The water outlet 11-13 has a smooth end surface 11-12-1. Because the sprayer 11 often needs to slide against the surface of the teeth or gingivae in the using process, the smooth end surface is designed to ensure that there is no accidentally damage to the teeth or gingivae in the using process, and the using process is safer and more comfortable.

The nozzle 11-1 has a radian 11-14 meeting the requirements of a human oral cavity. Especially, when it is necessary to clean the molars on the rear side along the outer side of the teeth, the radian 11-14 allows the sprayer 11 to smoothly reach the positions of the molars, so that the using process is more comfortable.

In the present embodiment, the water outlet 11-13 adopts a conical outlet which can be conveniently aligned to the tooth gap to be cleaned.

Referring to FIG. 7 to FIG. 9-2, the water outlet 11-13 may also have different shapes, such as an elliptical shape, a flat shape or a spherical shape. A user may select different outlet shapes according to the shape and area of the part to be cleaned. For example, the user may select a flat outlet for cleaning the joint between the teeth and the gingivae, and the using process is more convenient. Herein, the applicant only enumerates several shapes of the water outlets, and those skilled in the art may design the water outlets 11-13 of other various shapes according to needs without departing from the scope of protection of the present application.

Referring to FIG. 1-3 and FIG. 1-4, the connecting mechanism 11-2 includes a positioning block 31; the positioning block 31 is of an inverted T-shaped structure; the housing 21 of the oral observation instrument 200 is provided with a positioning groove 32 and a clamping block 33 capable of being connected to the connecting mechanism 11-2; and the positioning block 31 on the connecting mechanism 11-2 can be embedded in the positioning groove 32, and the clamping block 33 can prevent the connecting mechanism 11-2 from sliding backward, so that the sprayer 11 is fixed on the oral observation instrument 200 to realize connection between the sprayer 11 and the spray lance 12. The positioning block 31, the positioning groove 32 and the clamping block 33 cooperate to form the connecting mechanism 300.

The sprayer for a visual oral irrigator of the invention is connected to the spray lance 12 in a detachable mounting mode, so that after use, the sprayer 11 can be removed from the oral observation instrument 200 and then cleaned and replaced, which facilitates cleaning and makes the using process more sanitary.

### Embodiment 2: visual oral irrigator of the invention

Referring to FIG. 1 to FIG. 4-1, the visual oral irrigator of the present embodiment includes the sprayer 11 of Embodiment 1.

The visual oral irrigator 900 includes an oral irrigator 100, an oral observation instrument 200 and a connecting mechanism 300.

The oral irrigator 100 includes a sprayer 11, a spray lance 12, a control switch 13, a pressure vessel 14, a connecting pipe 15 and a connector 16; the sprayer 11 is detachably disposed at the front end of the spray lance 12; the control switch 13 may control the spraying of the fluid in the spray lance 12; and the fluid in the pressure vessel 14 is connected to the spray lance 12 through the connecting pipe 15 and the connector 16.

The oral observation instrument 200 includes a housing 21, a power system 22, an illuminating system 23, an observation system 24, a circuit system 25 and a switch 26; the illuminating system 23, the observation system 24, the circuit system 25 and the power system 22 are mounted in the housing 21, and the switch 26 is mounted on the housing 21; and the illuminating system 23, the observation system 24, the power system 22 and the switch 26 are connected together through the circuit system 25.

The oral irrigator 100 is mounted on the oral observation instrument 200 through the connecting mechanism 300.

The pressure vessel 14 of the oral irrigator 100 includes a pressurizing device 14-1 and a water tank 14-12; the water tank 14-12 includes a housing 14-2 and a fluid containing space 14-3; and the pressurizing device 14-1 can pressurize the fluid in the water tank 14-12.

In the present embodiment, the water tank 14-12 is detachably mounted on the connecting pipe 15 in a mode of interference fit combination. By virtue of a detachable connection mode, the water tank 14-12 can be conveniently removed from the connecting pipe 15 and then cleaned in the using process, so that the using process is more sanitary.

The connecting pipe 15 is provided with a connecting seat 15-3. The connecting seat 15-3 includes a gas inlet 15-31, a water outlet 15-32, a seal ring 15-33 and a base 15-34. The seal ring 15-33 is disposed on an outer ring of the base 15-34, and is integrally formed with the base 15-34. The housing 14-2 of the water tank 14-12 is provided with a wedge-shaped positioning block 14-21 and a slide groove 14-22. Referring to FIG. 4 and FIG. 4-1, the housing of the oral observation instrument 200 is provided with a positioning groove 21-1 and a slide rail 21-2.

When the water tank 14-12 is mounted, the slide groove 14-22 slides upward along the slide rail 21-2 until the wedge-shaped positioning block 14-21 is embedded into the positioning groove 21-1, and an elastic sheet 21-11 in the positioning groove 21-1 is bounced to prevent the wedge-shaped positioning block 14-21 from being released from the positioning groove 21-1. In this case, because the outer diameter of the seal ring 15-33 is greater than the inner diameter of a water filling hole 14-4 of the water tank 14-12, the soft seal ring 15-33 of the connecting seat 15-3 and the hard water filling hole 14-4 of the water tank 14-12 form interference fit to realize sealing after the water tank 14-12 is mounted, and connection between the water tank 14-12 and the connecting seat 15-3 is realized by concave-convex clamping of the wedge-shaped positioning block 14-21 and the positioning groove 21-1.

When the water tank 14-12 needs to be removed, the water tank 14-12 is forcibly pulled down, the elastic sheet 21-11 in the positioning groove 21-1 is pressed down, the wedge-shaped positioning block 14-21 is released from the positioning groove 21-1, and then, the water tank 14-12 can be removed.

The pressurizing device 14-1 may be a mechanical pressurizing device or an electric pressurizing device. In the present embodiment, the pressurizing device 14-1 is an electric pressurizing device 142.

The electric pressurizing device 142 can realize automatic pressurization by just switching on a power source and can stabilize the pressure, so that the using process is more convenient. When the pressurizing device 14-1 is the electric pressurizing device 142, the power system 22 may be provided with a pressurizing device 22-1 to increase the voltage of the power system 22, thereby better driving the electric pressurizing device 142 to work.

In the present embodiment, the electric pressurizing device 142 is an air electric pressurizing device 142-1, and the air electric pressurizing device 142-1 is an electric air compressor 142-11.

Referring to FIG. 1-2 and FIG. 2-1, the electric air compressor 142-11 includes a gas inlet 142-11-1, a gas filling pipe 142-11-2 and a compressor 142-11-3.

In the present embodiment, in order to better drive the electric air compressor 142-11 to work, the power system 22 is provided with the pressurizing device 22-1, a connecting mechanism 22-3 is disposed between the power source 22-2 of the power system 22 and the pressurizing device 22-1, and the pressurizing device 22-1 can pressurize the power system 22.

The electric air compressor 142-11 is mounted in the housing 21 of the oral observation instrument 200, and the gas filling pipe 142-11-2 is connected to the gas inlet 15-31 of the connecting seat 15-3. After being pressurized by the compressor 142-11-3, the air enters the water tank 14-12 from the gas inlet 15-31 of the connecting seat 15-3 through the gas filling pipe 142-11-2 so as to pressurize the fluid in the water tank 14-12. The pressurized fluid enters the connecting pipe 15 through the water outlet 15-32, enters the spray lance 12 through the connector 16, and finally is sprayed from the nozzle 11-1 of the sprayer 11 so as to rinse the part to be cleaned.

The electric air compressor 142-11 is mounted in the housing 21 of the oral observation instrument 200, the pressurized air is conveyed into the water tank 14-12 only through the gas filling pipe 142-11-2, and the fluid in the water tank 14-12 is pressurized to be effectively isolated from the water tank 14-12 so as to effectively prevent the fluid from entering the compressor 142-11-3, so that the using process is safer and more reliable.

The observation system 24 is a camera system 24-1, and the camera system 24-1 includes a camera 24-1-1, a data processing and output system 24-1-2, a circuit system 25 and a power system 22.

Referring to FIG. 13 and FIG. 14, video data output by the data processing and output system 24-1-2 of the camera system 24-1 can be displayed on a display 24-1-3 in a mode of wired connection or wireless connection, and the display 24-1-3 includes a smart phone 24-1-31, or a computer 24-1-32, or a liquid crystal display 24-1-33, or a television 24-1-34.

The illuminating system 23 is disposed at the periphery of the camera 24-1-1. In order to increase illumination, more LED lamps may also be disposed on the housing 21 of the oral observation instrument 200 so as to adjust the illumination brightness.

During working, when the switch 26 is turned on, the power system 22 and the electric air compressor 142-11 are switched on, the electric air compressor 142-11 works, the air is sucked into the electric air compressor 142-11 from the gas inlet 142-11-1, pressurized by the air compressor 142-11-3 and then introduced into the fluid containing space 14-3 of the pressure vessel 14 through the gas filling hole 142-11-21 of the gas filling pipe 142-11-2, thereby increasing the water pressure. When the control switch 13 is turned on, the pressurized water enters the spray lance 12 through the connecting pipe 15 disposed at the bottom of the fluid containing space 14-3 and then is sprayed from the sprayer 11 so as to clean the teeth.

In the present embodiment, a front end 900-1 of the visual oral irrigator 900 is connected to a main body 900-2 of the visual oral irrigator 900 through a foldable connecting mechanism 900-4.

In the present embodiment, the foldable connecting mechanism 900-4 is a rotating shaft type moving mechanism or a concave-convex clamping mechanism, or folding modes of other various structures may be designed according to needs within the scope of protection of the present application.

The foldable connecting mechanism 900-4 includes a rotating shaft 900-4-1 and a rotating shaft hole 900-4-2, and the rotating shaft 900-4-1 can move in the rotating shaft hole 900-4-2.

Referring to FIG. 5 to FIG. 6-1, a front end 200-1 of the oral observation instrument 200 of the visual oral irrigator 900 is connected to a main body 200-2 of the oral observation instrument 200 through a foldable connecting mechanism 900-4. Because a circuit 25-1 of the front end 200-1 of the oral observation instrument 200 is connected to a circuit 25-2 of the main body 200-2 of the oral observation instrument 200 through a bendable flexible circuit, after use, the front end 200-1 of the oral observation instrument 200 is rotated around the rotating shaft 900-4-1, and then, the front end 200-1 of the oral observation instrument 200 can be folded relative to the main body 200-2 of the oral observation instrument 200 to facilitate storage and carrying. When the oral observation instrument 200 needs to be used, the front end 200-1 of the oral observation instrument 200 is reversely rotated around the rotating shaft 900-4-1, so that the oral observation instrument 200 can be unfolded.

A front end 100-1 of the oral irrigator 100 of the visual oral irrigator 900 is connected to a main body 100-2 of the oral irrigator 100 through a foldable connecting mechanism 900-4. When the front end 100-1 of the oral irrigator 100 rotates around the rotating shaft 900-4-1 of the foldable connecting mechanism 900-4, the front end 100-1 of the oral irrigator 100 can be unfolded or folded relative to the main body 100-2 of the oral irrigator 100. A water path of the front end 100-1 of the oral irrigator 100 is connected to a water path of the main body 100-2 of the oral irrigator 100 through the connector 16. The connector 16 does not need to be repeatedly folded like a hose in a folding process, so that the fatigue fracture of the hose caused by repeated bending can be effectively avoided, thereby effectively prolonging the service life of the product.

In use, firstly, the front end 900-1 of the visual oral irrigator 900 is rotated around the foldable connecting mechanism 900-4 to unfold the visual oral irrigator 900, and then, the sprayer 11 is connected to the housing 21 of the oral observation instrument 200 through the connecting mechanism 11-2 to realize connection between the sprayer 11 and the spray lance 12. The oral observation instrument 200 is started, the water outlet 11-13 of the sprayer 11 is aligned to the tooth gap to be cleaned, the control switch 13 is turned on, and then the pressurized water is sprayed from the water outlet 11-13 to accurately clean the part to be cleaned. Furthermore, the cleaning process may be shot and photographed according to needs. When the water tank 14-2 needs to be cleaned, the water tank 14-2 is removed from the connecting seat 15-3 and then cleaned. After use, the sprayer 11 and the water tank 14-2 are removed and then cleaned thoroughly, the front end 900-1 of the visual oral irrigator 900 is rotated around the foldable connecting mechanism 900-4 to fold the visual oral irrigator 900 for storage.

Because the water outlet 11-13 of the visual oral irrigator 900 of the invention is within the field of view of the observation system 24, the position of the water outlet 11-13 and the flow rate, moving direction and moving speed of the fluid when sprayed from the water outlet 11-13 can be observed in real time, so as to really realize visual and accurate operation on the part to be cleaned. Furthermore, by adopting a detachable design mode for the visual oral irrigator of the invention, both the sprayer 11 and the water tank 14-2 can be conveniently removed and then cleaned, so that the using process is more sanitary and convenient.

### Embodiment 3: visual oral irrigator with threaded connection type water tank of the invention

Referring to FIG. 10 to FIG. 12, the difference between the present embodiment and the embodiment 2 lies in that: in the present embodiment, the water tank 14-12 is connected to the connecting seat 15-3 of the connecting pipe 15 in a mode of threaded connection.

Referring to FIG. 11-1 and FIG. 11-2, the outer side of the water filling hole 14-4 of the water tank 14-12 is provided with internal threads 14-41, and the base 15-34 of the connecting seat 15-3 is provided with external threads 15-34-1. When the water tank 14-12 is mounted, it is only necessary to rotate the water tank 14-12 to connect and tighten the internal threads 14-41 and the external threads 15-34-1, then the end of the water filling hole 14-4 of the water tank 14-12 abuts against the seal ring 15-33 of the connecting seat 15-3, and the seal ring 15-33 is pressed to form good sealing on the connecting part.

The threaded connection type water tank is simpler in mounting and removing processes, firmer in connection and safer and more convenient in using process.

In the present application, the applicant only specifically describes two connection modes of interference fit and threaded connection between the water tank 14-12 and the connecting pipe 15. Those skilled in the art may design the connection modes of other various structures to realize connection between the water tank 14-12 and the connecting pipe 15 without departing from the scope of protection of the present application.

It should be noted that the structures disclosed and described herein may be replaced with other structures having the same effect, and the embodiments described herein are not the only structures that can implement the invention. Although the preferred embodiments of the invention have been described herein, it is apparent to those skilled in the art that the embodiments are merely examples, and various variations, modifications, and replacements may be made by those skilled in the art without departing from the invention. Therefore, the protection scope of the invention should be subject to the spirit and scope of the appended claims of the invention.

## Claims

1. A sprayer for a visual oral irrigator, wherein the sprayer (11) comprises a nozzle (11-1) and a connecting mechanism (11-2);
A. the nozzle (11-1) comprises a water inlet (11-11), a channel (11-12) and a water outlet (11-13); the water inlet (11-11) is connected to an outlet (12-11) of a fluid channel (12-1) of a spray lance (12) of an oral irrigator (100) of a visual oral irrigator (900); a fluid in the oral irrigator (100) flows through the outlet (12-11) of the fluid channel (12-1) of the spray lance (12), then enters the water inlet (11-11), flows through the channel (11-12) and then is sprayed from the water outlet (11-13);
B. the nozzle (11-1) is detachably mounted on a housing (21) of an oral observation instrument (200) of the visual oral irrigator (900) through the connecting mechanism (11-2) to realize connection between the sprayer (11) and the spray lance (12).

2. The sprayer for a visual oral irrigator according to claim 1, wherein the water outlet (11-13) is within the field of view of an observation system (24) of the oral observation instrument (200).

3. The sprayer for a visual oral irrigator according to claim 1, wherein the water outlet (11-13) has a smooth end surface (11-12-1).

4. The sprayer for a visual oral irrigator according to claim 1, wherein the nozzle (11-1) has a radian (11-14) meeting the requirements of a human oral cavity.

5. The sprayer for a visual oral irrigator according to claim 1, wherein the water outlet (11-13) has different shapes, such as a conical shape, an elliptical shape, a flat shape or a spherical shape.

6. The sprayer for a visual oral irrigator according to claim 1, wherein the connecting mechanism (11-2) comprises a positioning block (31); the positioning block (31) is of an inverted T-shaped structure; the housing (21) of the oral observation instrument (200) is provided with a positioning groove (32) and a clamping block (33) capable of being connected to the connecting mechanism (11-2); and the positioning block (31) on the connecting mechanism (11-2) can be embedded in the positioning groove (32), and the clamping block (33) can prevent the connecting mechanism (11-2) from sliding backward, so that the sprayer (11) is fixed on the oral observation instrument (200) to realize connection between the sprayer (11) and the spray lance (12).

7. A visual oral irrigator, wherein an oral irrigator (100) of the visual oral irrigator (900) comprises the sprayer (11) according to claim 1.

8. The visual oral irrigator according to claim 7, wherein the visual oral irrigator (900) comprises an oral irrigator (100), an oral observation instrument (200) and a connecting mechanism (300);
A. the oral irrigator (100) comprises a sprayer (11), a spray lance (12), a control switch (13), a pressure vessel (14), a connecting pipe (15) and a connector (16); the sprayer (11) is detachably disposed at a front end of the spray lance (12); the control switch (13) is capable controlling the spraying of a fluid in the spray lance (12); the fluid in the pressure vessel (14) is connected to the spray lance (12) through the connecting pipe (15) and the connector (16);
B. the oral observation instrument (200) comprises a housing (21), a power system (22), an illuminating system (23), an observation system (24), a circuit system (25) and a switch (26); the illuminating system (23), the observation system (24), the circuit system (25) and the power system (22) are mounted in the housing (21), and the switch (26) is mounted on the housing (21); the illuminating system (23), the observation system (24), the power system (22) and the switch (26) are connected together through the circuit system (25);
C. the oral irrigator (100) is mounted on the oral observation instrument (200) through the connecting mechanism (300).

9. The visual oral irrigator according to claim 8, wherein the pressure vessel (14) of the oral irrigator (100) comprises a pressurizing device (14-1) and a water tank (14-12); the water tank (14-12) comprises a housing (14-2) and a fluid containing space (14-3); and the pressurizing device (14-1) can pressurize the fluid in the water tank (14-12).

10. The visual oral irrigator according to claim 9, wherein the water tank (14-12) is detachably mounted on the connecting pipe (15).

11. The visual oral irrigator according to claim 10, wherein the water tank (14-12) is connected to the connecting pipe (15) in a mode of threaded connection, or a mode of concave-convex clamping connection or a mode of interference fit connection.

12. The visual oral irrigator according to claim 8, wherein the observation system (24) is a camera system (24-1), and the camera system (24-1) comprises a camera (24-1-1), a data processing and output system (24-1-2), a circuit system (25) and a power system (22).

13. The visual oral irrigator according to claim 12, wherein video data output by the data processing and output system (24-1-2) of the camera system (24-1) can be displayed on a display (24-1-3) in a mode of wired connection or wireless connection, and the display (24-1-3) comprises a smart phone (24-1-31), or a computer (24-1-32), or a liquid crystal display (24-1-33), or a television (24-1-34).

14. The visual oral irrigator according to claim 8, wherein a front end (900-1) of the visual oral irrigator (900) is connected to a main body (900-2) of the visual oral irrigator (900) through a foldable connecting mechanism (900-4).

15. The visual oral irrigator according to claim 14, wherein the foldable connecting mechanism (900-4) is a rotating shaft type moving mechanism or a concave-convex clamping mechanism.

16. The visual oral irrigator according to claim 15, wherein the foldable connecting mechanism (900-4) is a rotating shaft type connecting mechanism, the foldable connecting mechanism (900-4) comprises a rotating shaft (900-4-1) and a rotating shaft hole (900-4-2), and the rotating shaft (900-4-1) can move in the rotating shaft hole (900-4-2).

17. The visual oral irrigator according to claim 14, wherein a front end (200-1) of the oral observation instrument (200) of the visual oral irrigator (900) is connected to a main body (200-2) of the oral observation instrument (200) through a foldable connecting mechanism (900-4).

18. The visual oral irrigator according to claim 14, wherein a front end (100-1) of the oral irrigator (100) of the visual oral irrigator (900) is connected to a main body (100-2) of the oral irrigator (100) through a foldable connecting mechanism (900-4).
